# EUROPÄISCHE PATENTSCHRIFT

(11) **EP 1 994 892 B1**
(45) Veröffentlichungstag und Bekanntmachung des Hinweises auf die Patenterteilung: **19.12.2012**
(21) Anmeldenummer: 08009307.3
(22) Anmeldetag: 20.05.2008
(51) Int. Cl.: A61B 17/17, A61B 17/34

(54) **Instrumentensatz zur minimal invasiven Vorbereitung einer Knochennagelung**
Instrument set for minimally invasive preparation of a bone nailing procedure
Ensemble d'instruments destiné à la préparation mini-invasive d'un clouage d'os

(30) Priorität: 23.05.2007 DE 202007007322 U
(43) Veröffentlichungstag der Anmeldung: 26.11.2008
(73) Patentinhaber: Baumgart, Rainer, 81479 München (DE)
(72) Erfinder: Baumgart, Rainer, 81479 München (DE)
(74) Vertreter: Hano, Christian

(56) Entgegenhaltungen:
- EP-A- 0 617 927
- EP-A- 1 340 467
- EP-A- 1 743 591
- WO-A-00/09024
- WO-A-01/60263
- WO-A2-2005/039651
- US-A1- 2006 200 160

## Beschreibung

Die Erfindung bezieht sich auf einen Instrumentensatz zur minimal invasiven Vorbereitung einer Knochennagelung.

Der Instrumentensatz ist in erster Linie für eine minimal invasive Vorbereitung einer Knochennagelung für Oberschenkel-, Unterschenkel- und Oberarmknochen vorgesehen. Grundsätzlich kann er aber auch zur minimal invasiven Vorbereitung einer Nagelung von anderen Knochen zum Einsatz kommen.

Im medizinisch-operativen Bereich sind zunehmend minimal invasive Verfahren gefragt. Dies betrifft auch die Unfallchirurgie und die Orthopädie. Ein seit Mitte des letzten Jahrhunderts etabliertes Verfahren ist die Knochennagelung. Hierbei wird ein solider oder innen hohler Metall-Stabilisator in den Markraum großer Röhrenknochen zur inneren Schienung eingebracht. Während früher hierzu große Schnitte erforderlich waren, wird zunehmend versucht, auch für dieses zukunftsträchtige OP-Verfahren minimal invasive, d.h. wenig gewebsschädigende Operationstechniken zu verwirklichen.

Stand der Technik sind einzelne Implantationshilfen, wie z.B. wannen- oder rohrförmige Gewebeschutzinstrumente, die mit einem Griff versehen sind und zum Schutz der Weichteile eingesetzt werden können. Bei kleinkalibrigen Marknägeln, die vorzugsweise in der Frakturbehandlung eingesetzt werden, bei denen keine Markraumbohrung erforderlich ist, lässt sich damit bereits gewebeschonend operieren.

Für großkalibrige Marknägel, die insbesondere für sekundäre Korrekturmaßnahmen vorteilhaft sind, aber auch zunehmend in der Frakturversorgung wieder Bedeutung gewinnen, in jedem Fall aber für Marknägel, die eine Konfektionierung des Markraumes erfordern, d.h. eine Fräsung gemäß Planung und nicht dem Weg des geringsten Widerstandes folgend, gibt es bisher keine Lösung, die den derzeitigen Ansprüchen genügt.

Bisher wird bei der Verwendung von Markraumfräsern ein Gewebeschutz in Form eines gebogenen Blechs oder in Form einer auf den Knochen aufgesetzten Hülse vorgesehen. Diese Werkzeuge weisen zahlreiche Nachteile auf. Bei einem Zugang zum Oberschenkelknochen von proximal beispielsweise verteilt sich das Abraummaterial bisher in den Weichteilen und führt vereinzelt zu störenden Ossifikationen. Bei Zugang zum Oberschenkelknochen von distal verteilt sich das Abraummaterial bisher im Kniegelenk, was ebenfalls nachteilig ist.

Wenn beispielsweise eine Knochenfehlstellung korrigiert und mit einem Marknagel stabilisiert werden soll, wird zunächst der Knochen operativ in ein eintrittsfernes und ein eintrittsnahes Knochensegment durchtrennt, sofern nicht bereits eine Kontinuitätsunterbrechung vorliegt. Die Auffräsung des eintrittsfernen Knochensegments in Schaftmitte muss aufgrund der meist harten diaphysären Knochenstruktur in vielen aufeinander folgenden Fräsvorgängen erfolgen. Da die Fräser bei jedem Wechsel das eintrittsnahe Knochensegment passieren und diese Passage weichteilbedingt oft unter einem ungünstigen Winkel erfolgt, entstehen bisher erhebliche Korrekturverluste durch sekundäre Fehlfräsungen im eintrittsnahen Knochensegment, siehe z.B. US-A-2006/200160.

In der EP 1 743 591 A2 ist eine Dilatationshülse offenbart, die an ihrem vorderen Ende einen sich konisch verjüngenden Abschnitt aufweist. In der Dilatationshülse ist eine Feder und eine Innenhülse angeordnet, die von einem Abtastsensor umgeben ist. Innerhalb der Innenhülse ist ein Bohrer angeordnet, der einen Schaft und eine Bohrspitze aufweist. Die Bohrspitze ist konisch ausgebildet, wobei ihr Außendurchmesser größer ist als der Innendurchmesser einer Bohrung in der Spitze der Dilatationshülse, so dass der Bohrer nicht mit seinem Schaft durch die Öffnung in der Spitze hindurch treten kann. Der Außendurchmesser des Schaftes des Bohrers ist deutlich geringer als der Innendurchmesser der Innenhülse. Die Innenhülse ist kürzer als die Dilatationshülse.

Die EP 1 340 467 A2 beschreibt die allgemein bekannte Technik, den Eintrittsort einer Dilatationshülse durch eine Führungsnadel festzulegen. Es ist ein System offenbart, das eine Führungsnadel, einen Dilator, ein weiches Rohr und ein Hülle für eine Operation umfasst. Der Dilator besteht aus einer Vielzahl von Rohren, die übereinander angeordnet sind. Bei der Verwendung diese Systems wird zunächst die Führungsnadel durch die Haut eingesetzt. Anschließend wird das erste Rohr des Dilators auf die Führungsnadel geschoben, bis seine Spitze einen bestimmten Punkt erreicht. Dann wird die Nadel herausgezogen und das nächste Rohr über das Rohr geschoben usw. Über das größte Rohr wird schließlich das weiche Rohr geschoben, in das dann die Hülle eingesetzt wird.

In der WO 2005/039651 A2 ist ein Instrumentensatz zur Vorbereitung eines Behandlungsortes im Rückgrat eines Patienten offenbart. Der Instrumentensatz umfasst einen starren Führungsdraht, der durch die Haut zu dem Behandlungsort geführt wird. Über den Führungsdraht wird zur Aufweitung eine sich konisch verjüngende Dilatationshülse eingeschoben. Auf die Dilationshülse wird dann eine Basishülse in Richtung des Behandlungsortes geschoben. Schließlich wird ein Fräswerkzeug in die Basishülse eingeführt, um den Behandlungsort auszufräsen. Die Basishülse dient auch dazu, ein nukleotomisches Instrument zur Bearbeitung einer Bandscheibe oder eine Extraktionsinstrument zur Extrahierung von Gewebe einzuführen.

Die der Erfindung zugrundeliegende Aufgabe besteht darin, einen Instrumentensatz bereitzustellen, mit dem es insgesamt möglich ist, einen minimal invasiven Zugangsweg zu realisieren, während des gesamten Vorgangs für eine weitgehende Weichteilschonung zu sorgen, eine Kontaminierung durch Abraummaterial zu vermeiden, die Nageleintrittsstelle präzise festzulegen, die Fräsrichtung und damit die Nagelrichtung präzise vorzugeben und für eine Aushülsung der Fräsbahn eines eintrittsnahen Knochensegments von zwei durch einen Nagel zu verbindenden Knochensegmenten zu sorgen.

Diese Aufgabe wird durch einen Instrumentensatz gemäβ Anspruch 1 zur minimal invasiven Vorbereitung einer Knochennagelung gelöst, der
a) wenigstens eine Basishülse mit einer ausgewählten axialen Länge, mit einem Eintreibende, mit einem Werkzeugangriffsende und mit einem für die Aufnahme von Arbeitshülsen oder eines Nagels, insbesondere Marknagels, ausgewählten Innendurchmesser,
b) wenigstens einen im Wesentlichen starren Führungsdraht mit einem ausgewählten Durchmesser und mit einer Fixierungsspitze an einem Ende,
c) wenigstens eine Dilatationshülse mit einem an den Innendurchmesser der Basishülse für eine geführte Verschiebung in ihr angepassten Außendurchmesser, mit einem sich von ihr weg verjüngenden Endabschnitt, der eine Auslassöffnung zum geführten Längsverschieben des Führungsdrahts durch sie hindurch aufweist, und mit einer Länge, die größer ist als die der Basishülse, und
d) wenigstens eine Arbeitshülse mit einem an den Innendurchmesser der Basishülse oder der nächst größeren Arbeitshülse für eine geführte Verschiebung in ihr angepassten Außendurchmesser, mit einer Länge, die größer ist als die der Basishülse, mit einem zur Führung eines Bohrers oder Fräsers bzw. eines zu setzenden Marknagels angepassten Innendurchmesser und mit einem Anschlag an ihrem einen Ende für den Eingriff mit dem Werkzeugangriffsende der Basishülse, aufweist.

Der Endabschnitt der Dilatationshülse ist vorteilhafterweise ein gerader oder schiefer Kreiskegel- oder Pyramidenstumpf. Bei der Ausführung als schiefer Kreiskegel- oder Pyramidenstumpf wird bei einem gesteuerten Drehen der Dilatationshülse um den Führungsdraht eine Positionskorrektur der Basishülse um den Betrag möglich, um den das spitzere Ende des schiefen Kreiskegel- oder Pyramidenstumpfes exzentrisch ausgeführt ist.

Vor allem dann, wenn der Endabschnitt der Dilatationshülse ein schiefer Kreiskegel- oder Pyramidenstumpf ist und eine Korrektur durch Drehen der Dilatationshülse herbeigeführt wird, neigt sie weichteilbedingt zu einem Zurückdrehen in die Ausgangslage. Da die Dilatationshülse am Ende nicht festgehalten werden kann, muss für eine Sicherung der neuen, verdrehten Position gesorgt werden. Dies wird durch einen in die Auslassöffnung verlängerten Fixierungsfortsatz erreicht, dessen Innendurchmesser für einen Schiebesitz mit dem Führungsdraht bemessen ist und dessen Außenfläche im Querschnitt die Form eines Mehrkants, beispielsweise eines Sechskants, hat. Wenn der so gestaltete Fixierungsfortsatz vollständig in den Knochen impaktiert ist, ist die durch Drehen eingestellte Position der Dilatationshülse gesichert und wird auch beim Einschlagen der Basishülse beibehalten.

Die beim Einschlagen der Basishülse auftretende Reaktionskraft hat die Tendenz, die Dilatationshülse zurückzutreiben, wodurch der Knochenkontakt und die durch Zentrierung zu dem Führungsdraht eingestellte Position verloren gehen könnte. Um den Außenmehrkant des Fixierungsfortsatzes in dem Knochen gegen ein Herausrutschen zu sichern, werden zweckmäßigerweise an seiner Außenfläche sich im Wesentlichen in Umfangsrichtung erstreckende, voneinander beabstandete Sicherungsvorsprünge vorgesehen.

Wenn die eintreibseitigen Flanken der Sicherungsvorsprünge zu ihren fortsatzfernen Enden hin von der Eintreibseite weg weisend abgeschrägt sind, wird das Eintreiben des Fixierungsfortsatzes erleichtert und aufgrund des hakenartigen Effekts ein Herausrutschen erschwert.

Die Sicherheitsvorsprünge können von Gewindegängen oder von in Radialebenen liegenden Umfangsrillen gebildet werden.

Zweckmäßigerweise ist das Werkzeugangriffsende der Basishülse ein sich von ihr radial nach außen erstreckender Ring, während der Anschlag an dem einen Ende der Arbeitshülse ein sich radial nach außen erstreckender Umfangswulst sein kann.

Der starre Führungsdraht hat zweckmäßigerweise einen Durchmesser von 2 bis 5 mm, vorzugsweise 3 mm.

Die Basishülse kann einen Innendurchmesser von 6 bis 20 mm, eine Wandstärke von 1 bis 3 mm haben und vorzugsweise aus Implantatstahl bestehen.

Der Instrumentensatz kann durch wenigstens ein Einschlaginstrument und/oder ein Extraktionswerkzeug für die Basishülse ergänzt werden.

Der Außendurchmesser der Arbeitshülsen ist auf den Innendurchmesser der Basishülsen oder der nächst größeren Arbeitshülse abgestimmt, so dass ein axiales Gleiten möglich ist. Die Innendurchmesser der Arbeitshülsen werden ausgehend von den in ihnen zu führenden Bohrern oder Fräsern sowie entsprechend dem Außendurchmesser des verwendeten Nagels, insbesondere Marknagels, bestimmt. Je nach Bedarf können auch mehrere im Durchmesser aufeinander abgestimmte Arbeitshülsen, auch unterschiedlicher Länge, in einer Basishülse platziert werden.

Für die Arbeitshülsen werden mehrere Längenabstufungen vorgesehen, um das geführte abgestufte Durchbohren von Knochensegmenten unterschiedlicher Abmessungen und das spätere genau geführte Setzen des Knochennagels zu ermöglichen. Die Längen der Basishülsen und Dilatationshülsen werden im Wesentlichen abhängig von den Dicken der zu durchdringenden Weichteile bestimmt.

Der Instrumentensatz besteht je nach Anspruch des Operateurs aus ca. 20 bis 100 Hülsen.

Anhand von Zeichnungen wird eine beispielsweise Ausführungsform der Erfindung näher erläutert. Es zeigt:
- Fig. 1: perspektivisch eine Basishülse, eine Dilatationshülse und einen Führungsdraht des Instrumentensatzes,
- Fig. 2: in einer Ansicht wie Fig. 1 eine Dilatationshülse mit einem Endabschnitt in Form eines schiefen Kreiskegelstumpfes,
- Fig. 3: in einer Ansicht wie Fig. 2 eine Dilatationshülse mit einem Endabschnitt in Form eines geraden Kreiskegelstumpfes und mit einem glatten Fixierungsfortsatz am verjüngten Ende des Endabschnitts der Dilatationshülse,
- Fig. 4: in einer Ansicht wie Fig. 3 den Fixierungsfortsatz mit Sicherungsvorsprüngen sowie in einer Einzelheit eine Ausgestaltung der Sicherungsvorsprünge,
- Fig. 5: perspektivisch auseinandergezogen eine Basishülse eingesetzt in einen Oberschenkelknochen von distal und eine Arbeitshülse beim Einsetzen in die Basishülse und
- Fig. 6: schematisch in einer teilweise geschnittenen Ansicht zwei Knochensegmente, in denen mit Hilfe einer Basishülse und von Arbeitshülsen, von denen nur eine gezeigt ist, eine ausgerichtete Bohrung für die anschließende Einführung eines Marknagels ausgebildet wird.

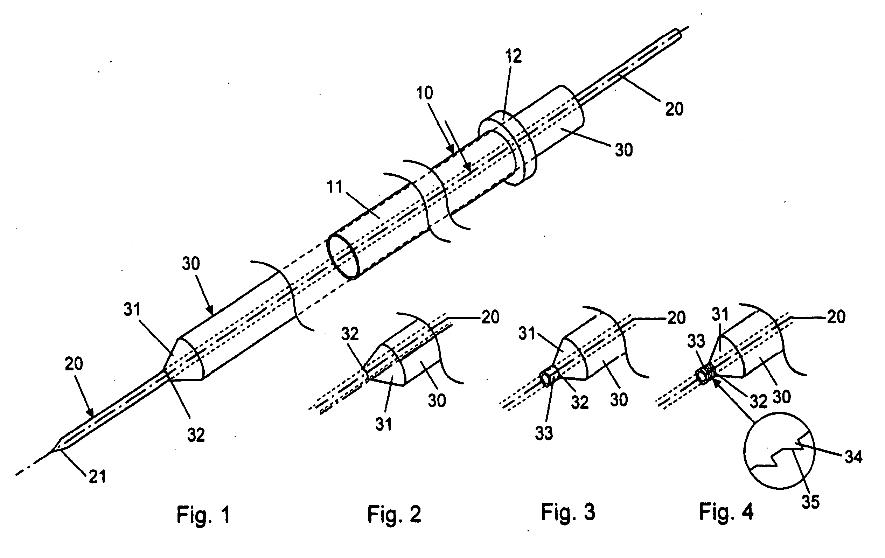

In Fig. 1 ist das zentrale Element des Instrumentensatzes gezeigt, das aus einer dünnwandigen Basishülse 10 mit einem Mantelteil 11 aus Stahl besteht, die an einem Ende einen sich radial nach außen erstreckenden Ring 12 für den Angriff mit einem Werkzeug aufweist. Der Instrumentensatz kann mehrere Basishülsen 10 mit Innendurchmessern zwischen 6 und 20 mm, mit Wandstärken zwischen 1 und 3 mm und mit unterschiedlichen einsatzabhängig vorgesehenen Längen aufweisen.

Fig. 1 zeigt eine Basishülse 10, die über eine Dilatationshülse 30 des Instrumentensatzes geschoben dargestellt ist. Die Dilatationshülse 30 hat an ihrem einen Ende einen sich verjüngenden Endabschnitt 31, der in einer Auslassöffnung 32 endet, die für den geführten Durchlass eines Führungsdrahtes 20 ausgebildet ist, der eine Spitze 21 hat.

Der sich verjüngende Endabschnitt 31 der Dilatationshülse 30 hat in der Ausführungsform von Fig. 1 die Form eines geraden Kreiskegelstumpfes. Bei der Ausführungsform von Fig. 2 hat der Endabschnitt 31 die Form eines schiefen Kreiskegelstumpfes.

Wie in Fig. 3 gezeigt ist, ist auf das verjüngte Ende des Endabschnitts 31 ein Fixierungsfortsatz 33 aufgesetzt, der einen zu der Auslassöffnung 32 des Endabschnitts 31 fluchtenden Durchgang mit einem Innendurchmesser aufweist, der dem Durchmesser der Auslassöffnung 32 entspricht und somit die geführte Verschiebung des Führungsdrahts 20 ermöglicht. Die Außenfläche des Fixierungsfortsatzes 33 bildet in der gezeigten Ausführung einen regelmäßigen Sechskant, an dessen Stelle auch andere Mehrkantprofile vorgesehen werden können.

Wie in Fig. 4 gezeigt ist, ist die Außenfläche des Fixierungsfortsatzes 33 mit Sicherungsvorsprüngen versehen, die in der an Fig. 4 im Kreis angefügten Einzelheit mit 34 bezeichnet sind und auf der Eintriebsseite abgeschrägte lange Flanken 35 haben, die sich von der Eintriebsseite weg zu ihrem fortsatzfernen Ende erstrecken und mit einer kurzen Flanke zur Außenfläche des Fixierungsfortsatzes zurückkehren.

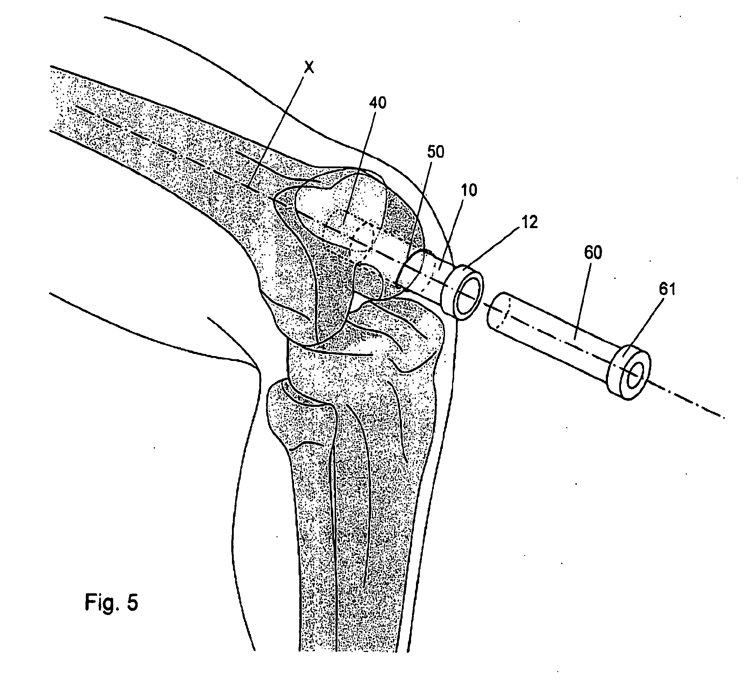

Fig. 5 zeigt eine Knochenanordnung, bei der eine Basishülse 10 durch eine Weichteilpassage 50 hindurch mit einem Fixierungsabschnitt 40 in einen Knochen entsprechend einer Achse X ausgerichtet eingetrieben ist. Fig. 5 zeigt ferner das Einführen einer Arbeitshülse 60 in die Basishülse 10, die in der vollständig eingeführten Stellung (nicht gezeigt) mit einem ringförmigen Wulst 61 an dem Ring 12 der Basishülse 10 stirnseitig anliegt.

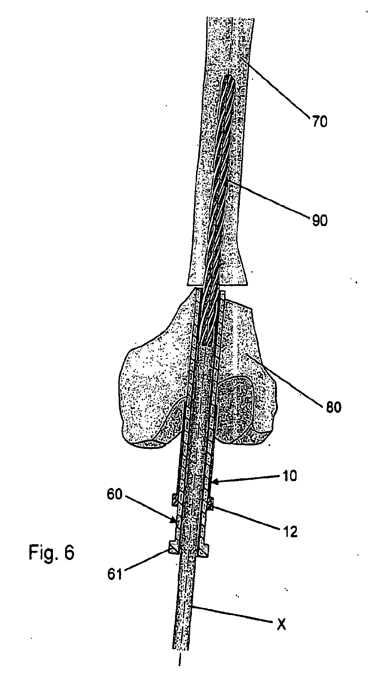

Fig. 6 zeigt zwei Knochensegmente 70 und 80, die längs einer Achse X zueinander ausgerichtet sind. Zur Vorbereitung ihrer Verbindung durch einen nicht gezeigten Nagel ist in dem Knochensegment 80 die Basishülse 10 fixiert, durch die eine Arbeitshülse 60 hindurch angeordnet ist, durch die hindurch wiederum ein Bohrer 90 so geführt ist, dass er eine zur Achse X ausgerichtete Bohrung im Knochensegment 70 ausbildet, wobei das Knochensegment 80 durch die Arbeitshülse 60 vollständig ausgehülst und damit geschützt ist.

Im Folgenden wird die Verwendung der Instrumente des Instrumentensatzes beispielsweise beschrieben.

Die Positionierung der Basishülse 10 sowohl hinsichtlich der Position zum Knochen als auch hinsichtlich der Ausrichtung zur Knochenachse ist von elementarer Bedeutung. Um die Position exakt zu bestimmen, wird der Eintrittspunkt mit einem eine Spitze 21 aufweisenden Führungsdraht 20 durch die Haut oder über einen Minischnitt unter einem Bildwandler festgelegt. Der Führungsdraht 20 besteht aus einem starren Material und hat gewöhnlich einen Durchmesser von 3 mm. Bei der Fixierung des Eintrittspunkts ist zunächst die Verlaufsrichtung in dem Knochen von untergeordneter Bedeutung.

Die Aufweitung der Weichteile im elastischen Bereich erfolgt über eine Dilatationshülse 30, die einen sich von ihr weg zu einer Auslassöffnung verjüngenden Endabschnitt 31 aufweist und die mit ihrer Auslassöffnung über den in Position gebrachten Führungsdraht 20 geschoben wird. Der Außendurchmesser der Dilatationshülse 30 und der Innendurchmesser der verwendeten Basishülse 10 sind so bemessen, dass die Basishülse 10 auf der Dilatationshülse 30 geführt verschiebbar ist.

Wenn der Endabschnitt 31 ein schiefer Kegel (Fig. 2) ist, lässt sich durch Drehen der Dilatationshülse 30 die Außenkontur der Dilatationshülse 30 gegenüber dem Führungsdraht 20 und damit die Arbeitshülse 10 in eine eventuelle Korrekturposition bringen, ohne dass der Führungsdraht 20 neu platziert werden muss. An der Dilatationshülse 30 sind dafür am äußeren freien Ende auf die Endkonusstellung ausgerichtete Markierungen angebracht.

Nach Fixierung des Eintrittspunkts mit Hilfe des Führungsdrahts 20 und nach Aufweitung der Weichteile mit Hilfe der über den Führungsdraht 20 geschobenen Dilatationshülse 30, die für die Positionierung der Basishülse 10 ausgerichtet wird, wird die Dilatationshülse 30 mit Hilfe ihres Fixierungsfortsatzes 33 dadurch fixiert, dass dieser in den Knochen eingetrieben wird, in welchem er durch seine Mehrkantaußenfläche (Fig. 3) gegen ein Verdrehen und durch das Rillenprofil gemäß Fig. 4 gegen ein Herausrutschen gesichert ist. Anschließend wird die Basishülse 10 über die Dilatationshülse 30 geschoben und bis zum Knochen, wie in Fig. 5 gezeigt ist, vorgetrieben. Dann wird sie mit dosierten Hammerschlägen unter Verwendung eines rohrförmigen Einschlagwerkzeuges in den Knochen unter Bildung eines Fixierungsabschnitts 40 impaktiert, wobei bei diesem Vorgang die geplante Fräsrichtung genau beachtet ist. In Abhängigkeit von der Knochenbeschaffenheit genügt es für eine sichere Richtungsvorgabe, dass der Fixierungsabschnitt 40 wenige Millimeter bis zu einem Zentimeter tief eingetrieben wird. Anschließend werden die Dilatationshülse 30 und der Führungsdraht 20 extrahiert.

Mit Abschluss dieser Maßnahme ist die spätere Marknageleintrittsstelle festgelegt und der Verlauf aller Bohrer oder Fräser und somit des späteren Marknagels im eintrittsnahen Knochensegment im Knochen definiert. Dafür ist nur eine einmalige Weichteilpassage 50 (Fig. 5) der Basishülse 10 erforderlich, die mit Hilfe der Dilatationshülse 30 erreicht wird, was hinsichtlich einer bakteriellen Kontamination vorteilhaft ist. Außerdem werden kleinere Blutungen in den Weichteilen komprimiert. Durch die in den Knochen impaktierte Basishülse 10, die für die gesamte Vorbereitung des Knochens für die Knochennagelung verbleibt, wird das gesamte Abraummaterial, das bei den Bohr- und Fräsvorgängen entsteht, nach außen geleitet und entsorgt.

In der Regel muss der Knochen - in Fig. 6 sind beispielsweise ein eintrittsnahes Knochensegment 80 und ein eintrittsfernes Knochensegment 70 gezeigt - konzentrisch stufenweise aufgefräst werden, wobei mit einem kleinen Durchmesser begonnen wird. Um eine stufenweise Reduktion des Innendurchmessers der Basishülse 10 auf den jeweiligen Fräserdurchmesser zu ermöglichen, werden die Arbeitshülsen 60 vorgesehen, die an ihrem einen Ende den radial nach außen stehenden ringförmigen Anschlag bzw. Wulst 61 aufweisen, für den der sich radial nach außen von der Basishülse 10 erstreckende Anschlag 12 in Form eines Rings als Widerlager dient. Der Außendurchmesser der Arbeitshülse 60 ist an den Innendurchmesser der jeweiligen Basishülsen 10 des Instrumentensatzes so angepasst, dass jede Arbeitshülse 60 in der zugehörigen Basishülse 10 axial und radial geführt verschiebbar ist.

Insbesondere wenn Achsenkorrekturen erforderlich sind, muss die Fräsrichtung in den trompetenförmigen Aufweitungen der Knochenenden exakt entsprechend Planungsvorgaben ausgeführt werden (Fig. 6). Erst dann kann die Auffräsung der mittleren Knochenregion erfolgen. Da die Knochenbohrer bzw. -fräser 90 hierzu wiederholt das eintrittsnahe Knochensegment 80 unter einem ungünstigen Winkel passieren müssen, kann durch den Einsatz entsprechend längerer Arbeitshülsen 60 das eintrittsnahe Knochensegment 80 auf der gesamten Länge geschient werden, so dass die Fräser 90 hier keinen Knochenkontakt haben. Durch entsprechenden Austausch der Arbeitshülsen 60 mit entsprechender Länge und an den Bohrer bzw. Fräser 90 angepassten Innendurchmessern wird eine konzentrische Ausfräsung des eintrittsfernen Knochensegments 70 gewährleistet, ohne dass hierbei ein Korrekturverlust in dem eintrittsnahen Knochensegment erfolgt. Es können hierbei auch mehrere Arbeitshülsen ineinander gesteckt werden. Wenn die Fräsung des eintrittsfernen Knochensegments in mehreren Schritten erfolgt und zugleich eine Aushülsung des eintrittsnahen Knochensegments zu dessen Schutz erforderlich ist, kann in die Anordnung so wie in Fig. 6 dargestellt, in die Arbeitshülse 60 auch eine oder mehrere Arbeitshülsen, im Durchmesser abgestimmt, eingeführt werden, so dass auch Bohrer oder Fräser mit kleineren Durchmessern konzentrisch geführt werden.

Nach Entfernen des zuletzt eingesetzten Bohrers 90 ist die minimal invasive Vorbereitung für die Knochennagelung abgeschlossen. Nun kann ein nicht gezeigter Marknagel durch die Basishülse hindurch in die fluchtend ausgerichteten Bohrungen in dem eintrittsnahen Knochensegment 80 und eintrittsfernen Knochensegment 70 positionsgerecht platziert werden. Die Basishülse wird anschließend entfernt.

Die Basishülse kann aber auch nach Abschluss der Vorbereitung zur Knochennagelung entfernt werden, so dass der Marknagel dann direkt in die fluchtend ausgerichteten Knochenbohrungen positioniert wird.

## Patentansprüche

1. Instrumentensatz zur minimal invasiven Vorbereitung einer Knochennagelung mit
a) wenigstens einer Basishülse (10), die
- eine ausgewählte axiale Länge mit einem Eintreibende und einem Werkzeugangriffsende (12) und
- einen für die Aufnahme von Arbeitshülsen (60) oder eines Nagels, insbesondere Marknagels, ausgewählten Innendurchmesser aufweist,
b) wenigstens einem im Wesentlichen starren Führungsdraht (20), der
- einen ausgewählten Durchmesser und
- an einem Ende eine Fixierungsspitze (21) aufweist,
**dadurch gekennzeichnet, daß** den Instrumentensatz ferner aufweist :
c) wenigstens eine Dilatationshülse (30), die
- einen an den Innendurchmesser der Basishülse (10) für eine geführte Verschiebung in ihr angepassten Außendurchmesser,
- einen sich von ihr weg verjüngenden Endabschnitt (31) mit einer Auslassöffnung (32) zum geführten Längsverschieben des Führungsdrahts (20) durch sie hindurch und
- eine Länge aufweist, die größer ist als die der Basishülse (10), und
d) wenigstens eine Arbeitshülse (60), die
- einen an den Innendurchmesser der Basishülse (10) oder der nächst größeren Arbeitshülse für eine geführte Verschiebung in ihr angepassten Außendurchmesser,
- eine Länge, die größer ist als die der Basishülse (10),
- einen zur Führung eines Bohrers oder Fräsers (90) bzw. eines zu setzenden Nagels angepassten Innendurchmesser und
- an ihrem einen Ende einen Anschlag (61) für den Angriff mit dem Werkzeugangriffsende (12) der Basishülse (10) aufweist.

2. Instrumentensatz nach Anspruch 1, **dadurch gekennzeichnet, dass** der Endabschnitt (31) der Dilatationshülse (30) ein gerader oder schiefer Kreiskegel- oder Pyramidenstumpf ist.

3. Instrumentensatz nach Anspruch 1 oder 2, **gekennzeichnet durch** einen die Auslassöffnung (32) der Dilatationshülse (30) verlängernden Fixierungsfortsatz (33), dessen Innendurchmesser für einen Schiebesitz mit dem Führungsdraht (20) bemessen ist und dessen Außenfläche im Querschnitt eine Mehrkantform hat.

4. Instrumentensatz nach Anspruch 3, **gekennzeichnet durch** an der Außenfläche des Fixierungsfortsatzes (33) sich im Wesentlichen in Umfangsrichtung erstreckende, voneinander beabstandete Sicherungsvorsprünge (34).

5. Instrumentensatz nach Anspruch 4, **dadurch gekennzeichnet, dass** die Sicherungsvorsprünge (34) eintreibseitige Flanken (35) aufweisen, die zu ihren fortsatzfernen Enden hin von der Eintreibseite wegweisend abgeschrägt sind.

6. Instrumentensatz nach Anspruch 4 oder 5, **dadurch gekennzeichnet, dass** die Sicherungsvorsprünge (34) von Gewindegängen oder von in Radialebenen liegenden Umfangsrillen gebildet werden.

7. Instrumentensatz nach einem der vorhergehenden Ansprüche, **dadurch gekennzeichnet, dass** das Werkzeugangriffsende (12) der Basishülse (10) von einem sich radial nach außen erstreckenden Ring gebildet wird.

8. Instrumentensatz nach einem der vorhergehenden Ansprüche, **dadurch gekennzeichnet, dass** der Anschlag (61) an dem einen Ende der Arbeitshülse (60) von einem sich radial nach außen erstreckenden Umfangswulst gebildet wird.

9. Instrumentensatz nach einem der vorhergehenden Ansprüche, **dadurch gekennzeichnet, dass** der Führungsdraht (20) einen Durchmesser von 2 bis 5 mm, vorzugsweise 3 mm hat.

10. Instrumentensatz nach einem der vorhergehenden Ansprüche, **dadurch gekennzeichnet, dass** die Basishülse (10) einen Innendurchmesser von 6 bis 20 mm hat.

11. Instrumentensatz nach einem der vorhergehenden Ansprüche, **dadurch gekennzeichnet, dass** die Wandstärke der Basishülse (10) 0,5 bis 3 mm beträgt.

12. Instrumentensatz nach einem der vorhergehenden Ansprüche, **dadurch gekennzeichnet, dass** wenigstens die Basishülse (10) aus Implantatstahl besteht.

13. Instrumentensatz nach einem der vorhergehenden Ansprüche, **dadurch gekennzeichnet, dass** zusätzlich wenigstens ein Einschlaginstrument und/oder Extraktionswerkzeug für die Basishülse (10) vorgesehen sind.

## Claims

1. An instrument set for a minimally invasive preparation for a bone nailing, comprising
a) at least one base sleeve (10) having
- a selected axial length with a drive-in end and a tool engaging end (12) and
- an inner diameter selected for receiving working sleeves (60) or a nail, in particular a medullary nail,
b) at least one substantially rigid guide wire (20) having
- a selected diameter and
- a fixing tip (21) at one end,
**characterized in that** the instrument set further comprises:
c) at least one dilation sleeve (30) having
- an outer diameter adapted to the inner diameter of the base sleeve (10) for a guided displacement therein,
- an end section (31) tapering away therefrom with an outlet opening (32) for the guided longitudinal displacement of the guide wire (20) therethrough, and
- a length which is greater than that of the base sleeve (10), and
d) at least one working sleeve (60) having
- an outer diameter adapted to the inner diameter of the base sleeve (10) or the next largest working sleeve for a guided displacement therein,
- a length which is greater than that of the base sleeve (10),
- an inner diameter adapted for guiding a drill or miller (90) or a nail to be inserted, and
- a stop (61) at its one end for engaging the tool engaging end (12) of the base sleeve (10).

2. The instrument set according to claim 1, **characterised in that** the end section (31) of the dilation sleeve (30) is a right or oblique truncated circular cone or pyramid.

3. The instrument set according to claim 1 or 2, **characterised by** a fixing extension (33) lengthening the outlet opening (32) of the dilation sleeve (30), the inner diameter of which is dimensioned for a sliding seat with the guide wire (20) and the outer surface of which has a polygonal cross-section.

4. The instrument set according to claim 3, **characterised by** securing projections (34) extending substantially in the peripheral direction and spaced apart from each other on the outer surface of the fixing extension (33).

5. The instrument set according to claim 4, **characterised in that** the securing projections (34) have flanks (35) on the drive-in side which are chamfered towards their ends remote from the extension and pointing away from the drive-in side.

6. The instrument set according to claim 4 or 5, **characterised in that** the securing projections (34) are formed by thread turns or by peripheral grooves lying in radial planes.

7. The instrument set according to any one of the preceding claims, **characterised in that** the tool engaging end (12) of the base sleeve (10) is formed by a radially outwardly extending ring.

8. The instrument set according to any one of the preceding claims, **characterised in that** the stop (61) at the one end of the working sleeve (60) is formed by a radially outwardly extending peripheral bead.

9. The instrument set according to any one of the preceding claims, **characterised in that** the guide wire (20) has a diameter of 2 to 5 mm, preferably 3 mm.

10. The instrument set according to any one of the preceding claims, **characterised in that** the base sleeve (10) has an inner diameter of 6 to 20 mm.

11. The instrument set according to any one of the preceding claims, **characterised in that** the wall thickness of the base sleeve (10) is 0.5 to 3 mm.

12. The instrument set according to any one of the preceding claims, **characterised in that** the base sleeve (10) is made of implant steel.

13. The instrument set according to any one of the preceding claims, **characterised in that** additionally at least one knock-in instrument and/or extraction tool for the base sleeve (10) is provided.

## Revendications

1. Ensemble d'instruments pour la préparation mini-invasive d'un clouage d'os, comprenant
a) au moins un manchon de base (10) qui
- présente une longueur axiale choisie avec une extrémité d'enfoncement et une extrémité de contact avec un outil (12) et
- un diamètre intérieur choisi pour la réception de manchons de travail (60) ou d'un clou, notamment un clou médullaire,
b) au moins un fil de guidage (20) essentiellement rigide qui
- présente un diamètre choisi et
- est pourvu d'une pointe de fixation (21) à une extrémité,
**caractérisé en ce que** l'ensemble d'instruments comprend, en outre :
c) au moins un manchon de dilatation (30) qui
- présente un diamètre extérieur adapté au diamètre intérieur du manchon de base (10) pour un déplacement guidé dans celui-ci,
- une portion d'extrémité (31) allant se rétrécissant en s'éloignant du manchon, pourvue d'une ouverture de sortie (32) pour le déplacement longitudinal guidé du fil de guidage (20) à travers l'ouverture et
- une longueur qui est supérieure à celle du manchon de base (10) et
d) au moins un manchon de travail (60) qui
- présente un diamètre extérieur adapté au diamètre intérieur du manchon de base (10) ou du manchon de travail de dimension immédiatement supérieure pour un déplacement guidé dans celui-ci,
- une longueur supérieure à celle du manchon de base (10),
- un diamètre intérieur adapté au guidage d'une perceuse ou fraiseuse (90) resp. d'un clou à mettre en place et,
- à une extrémité, une butée (61) pour le contact avec l'extrémité de contact avec un outil (12) du manchon de base (10).

2. Ensemble d'instruments selon la revendication 1, **caractérisé en ce que** la portion d'extrémité (31) du manchon de dilatation (30) est un cône de révolution ou pyramide à troncature droite ou oblique.

3. Ensemble d'instruments selon la revendication 1 ou 2, **caractérisé par** un appendice de fixation (33) prolongeant l'ouverture de sortie (32) du manchon de dilatation (30), dont le diamètre intérieur est dimensionné pour un siège coulissant avec le fil de guidage (20) et dont la surface extérieure a une section transversale de forme polygonale.

4. Ensemble d'instruments selon la revendication 3, **caractérisé par** des saillies de sûreté (34) situées à une distance les unes des autres et s'étendant essentiellement dans la direction circonférentielle sur la surface extérieure de l'appendice de fixation (33).

5. Ensemble d'instruments selon la revendication 4, **caractérisé en ce que** les saillies de sûreté (34) présentent des flancs (35), côté enfoncement, qui sont biseautés de manière à s'éloigner du côté d'enfoncement en allant vers leurs extrémités éloignées de l'appendice.

6. Ensemble d'instruments selon la revendication 4 ou 5, **caractérisé en ce que** les saillies de sûreté (34) sont formées par des pas de vis ou par des nervures circonférentielles situées dans des plans radiaux.

7. Ensemble d'instruments selon l'une des revendications précédentes, **caractérisé en ce que** l'extrémité de contact avec un outil (12) du manchon de base (10) est formée par une bague s'étendant radialement vers l'extérieur.

8. Ensemble d'instruments selon l'une des revendications précédentes, **caractérisé en ce que** la butée (61) est formée par un bourrelet circonférentiel s'étendant radialement vers l'extérieur à une extrémité du manchon de travail (60).

9. Ensemble d'instruments selon l'une des revendications précédentes, **caractérisé en ce que** le fil de guidage (20) a un diamètre compris entre 2 mm et 5 mm, de préférence de 3 mm.

10. Ensemble d'instruments selon l'une des revendications précédentes, **caractérisé en ce que** le manchon de base (10) a un diamètre intérieur compris entre 6 mm et 20 mm.

11. Ensemble d'instruments selon l'une des revendications précédentes, **caractérisé en ce que** l'épaisseur de paroi du manchon de base (10) est comprise entre 0,5 mm et 3 mm.

12. Ensemble d'instruments selon l'une des revendications précédentes, **caractérisé en ce qu'**au moins le manchon de base (10) est en acier pour implant.

13. Ensemble d'instruments selon l'une des revendications précédentes, **caractérisé en ce qu'**au moins un instrument de percussion et/ou un outil d'extraction pour le manchon de base (10) est/sont, en plus, prévu/s.
